# EUROPÄISCHE PATENTSCHRIFT

(11) **EP 1 917 323 B1**
(45) Veröffentlichungstag und Bekanntmachung des Hinweises auf die Patenterteilung: **18.12.2019**
(21) Anmeldenummer: 06762829.7
(22) Anmeldetag: 26.07.2006
(51) Int. Cl.: C09K 11/06, C07C 13/62, C07C 13/72, C07C 25/22, C07D 237/26, C07D 403/10, C07D 209/94, C07D 409/10, C07D 407/10, C07D 235/02, C07D 249/16, C07D 307/77, C07D 285/14, C07D 271/12, C07D 401/10

(54) **NEUE MATERIALIEN FÜR ORGANISCHE ELEKTROLUMINESZENZVORRICHTUNGEN**
NOVEL MATERIALS FOR ORGANIC ELECTROLUMINESCENT DEVICES
NOUVELLES MATIERES POUR DES DISPOSITIFS ELECTROLUMINESCENTS ORGANIQUES

(30) Priorität: 26.08.2005 DE 102005040411
(43) Veröffentlichungstag der Anmeldung: 07.05.2008
(60) Teilanmeldung: 17165036.9
(73) Patentinhaber: Merck Patent GmbH, 64293 Darmstadt (DE)
(72) Erfinder: HEIL, Holger, 64295 Darmstadt (DE); BUESING, Arne, 65929 Frankfurt (DE); STOESSEL, Philipp, 60487 Frankfurt (DE); VESTWEBER, Horst, 34630 Gilersberg-Winterscheid (DE); PARHAM, Amir, 65929 Frankfurt (DE)
(86) Internationale Anmeldenummer: PCT/EP2006/007386
(87) Internationale Veröffentlichungsnummer: WO 2007/022845

(56) Entgegenhaltungen:
- EP-A- 1 491 568
- EP-A1- 1 589 089
- JP-A- 2000 003 790
- LE BERRE, ANDRE: "Autoxidation of orthoquinoid indenofluorene hydrocarbons" ANN. CHIM., Bd. 2, 1957, Seiten 371-425, XP009074391 Paris
- HARVEY R G ET AL: "NEW SYNTHETIC APPROACH TO POLYCYCLIC AROMATIC HYDROCARBONS AND THEIR CARCINOGENIC OXIDEZED METABOLITES: DERIVATIVES OF BENZO[S]PICENE, BENZO[RST]PENTAPHENE, AND DIBENZO[B,DEF]CHRYSENE" JOURNAL OF ORGANIC CHEMISTRY, AMERICAN CHEMICAL SOCIETY. EASTON, US, Bd. 65, Nr. 13, 2000, Seiten 3952-3960, XP002271036 ISSN: 0022-3263 in der Anmeldung erwähnt
- DAHLMANN AND R NEIDLEIN U: "The diyne reaction of 3,3'-bis(phenylethynyl)-2,2'-bithiophene derivatives via rhodium complexes. A novel approach to condensed benzo[2,1-b:3,4-b'] dithiophenes", HELVETICA CHIMICA ACTA, VERLAG HELVETICA CHIMICA ACTA, CH, vol. 80, no. 1, 1 January 1997 (1997-01-01), pages 111-120, XP008139779, ISSN: 0018-019X

## Beschreibung

Organische Halbleiter werden für eine Reihe verschiedenartiger Anwendungen, die im weitesten Sinne der Elektronikindustrie zugerechnet werden können, entwickelt. Der Aufbau organischer Elektrolumineszenzvorrichtungen (OLEDs), in denen diese organischen Halbleiter als funktionelle Materialien eingesetzt werden, ist beispielsweise in US 4539507, US 5151629, EP 0676461 und WO 98/27136 beschrieben. Allerdings zeigen diese Vorrichtungen noch erhebliche Probleme, die einer dringenden Verbesserung bedürfen:
1. Die operative Lebensdauer ist insbesondere bei blauer Emission immer noch gering, so dass bis dato nur einfache Anwendungen kommerziell realisiert werden konnten.
2. Die verwendeten Verbindungen sind teilweise nur schwer in gängigen organischen Lösemitteln löslich, was ihre Reinigung bei der Synthese, aber auch die Reinigung der Anlagen bei der Herstellung der organischen elektronischen Vorrichtungen erschwert.
3. Einige der verwendeten Verbindungen, die ansonsten gute Eigenschaften in OLEDs zeigen, weisen keine ausreichend hohe Glasübergangstemperatur auf.

In fluoreszierenden OLEDs werden gemäß dem Stand der Technik verschiedene kondensierte Aromaten, insbesondere Anthracen- oder Pyrenderivate, als Host-Materialien vor allem für blau emittierende Elektrolumineszenzvorrichtungen verwendet, z. B. 9,10-Bis(2-naphthyl)-anthracen (US 5935721). In WO 03/095445 und in CN 1362464 werden 9,10-Bis(1-naphthyl)anthracen-Derivate für die Verwendung in OLEDs beschrieben. Weitere Anthracen-Derivate, die sich als Host-Materialien eignen, sind in WO 01/076323, in WO 01/021729, in WO 04/013073, in WO 04/018588, in WO 03/087023 oder in WO 04/018587 beschrieben. Host-Materialien, basierend auf Aryl-substituierten Pyrenen und Chrysenen, werden in WO 04/016575 beschrieben. Es ist für hochwertige Anwendungen notwendig, verbesserte Host-Materialien zur Verfügung zu haben.

Als Stand der Technik bei blau emittierenden Verbindungen kann die Verwendung bestimmter Arylvinylamine von Idemitsu genannt werden (z. B. WO 04/013073, WO 04/016575, WO 04/018587). Damit werden sehr gute Lebensdauern bei tiefblauer Emission zitiert. Allerdings sind diese Ergebnisse stark abhängig vom verwendeten Hostmaterial, so dass die zitierten Lebensdauern nicht als Absolutwerte verglichen werden können, sondern immer nur bei Einsatz in einem optimierten System. Weiterhin sind diese Verbindungen thermisch instabil und lassen sich nicht unzersetzt verdampfen, was daher einen hohen technischen Aufwand für die OLED-Herstellung erfordert und somit einen deutlichen technischen Nachteil darstellt. Einen weiteren Nachteil stellt die Emissionsfarbe dieser Verbindungen dar. Während Idemitsu tiefblaue Emission (CIE-y-Koordinaten im Bereich von 0.15-0.18) zitiert, konnten diese Farbkoordinaten nicht in einfachen Vorrichtungen gemäß dem Stand der Technik reproduziert werden. Im Gegenteil erhält man hier grünblaue Emission. Es ist nicht offensichtlich, wie mit diesen Verbindungen tatsächlich blaue Emission erzeugt werden kann. Es ist für hochwertige Anwendungen notwendig, verbesserte Emitter besonders im Bezug auf Device- und Sublimationsstabilität zur Verfügung zu haben.

In EP 1491568 A1 werden Oligomere und Polymere enthaltend cis-Indenofluoren-Einheiten als funktionelle Materialien zur Verwendung in OLEDs offenbart, unter anderem zur Verwendung in der emittierenden Schicht.

In phosphoreszierenden OLEDs wird als Matrixmaterial häufig 4,4'-Bis-(N-carbazolyl)biphenyl (CBP) verwendet. Die Nachteile sind unter anderem kurze Lebensdauern der mit ihnen hergestellten Devices und häufig hohe Betriebsspannungen, die zu geringen Leistungseffizienzen führen. Außerdem weist CBP eine nicht ausreichend hohe Glasübergangstemperatur auf. Des Weiteren hat sich gezeigt, dass CBP für blau emittierende Elektrolumineszenzvorrichtungen ungeeignet ist, was in einer schlechten Effizienz resultiert. Außerdem ist der Aufbau der Devices komplex, wenn CBP als Matrixmaterial verwendet wird, da zusätzlich eine Lochblockierschicht und eine Elektronentransportschicht verwendet werden müssen. Verbesserte Triplett-Matrixmaterialien, basierend auf Ketoverbindungen von Spirobifluoren, sind in WO 04/093207 beschrieben. Für die besten der dort beschriebenen Matrixmaterialien werden jedoch in der Synthese giftige anorganische Cyanide benötigt, so dass die Herstellung dieser Materialien ökologisch bedenklich ist. Von anderen der dort beschriebenen Matrixmaterialien ist die Glasübergangstemperatur noch nicht zufriedenstellend.

Als Elektronentransportverbindung in organischen Elektrolumineszenzvorrichtungen wird meist AlQ₃ (Aluminium-tris-hydroxychinolinat) verwendet (US 4539507). Dieses hat mehrere Nachteile: Es lässt sich nicht rückstandsfrei aufdampfen, da es sich bei der Sublimationstemperatur teilweise zersetzt, was insbesondere für Produktionsanlagen ein großes Problem darstellt. Ein weiterer Nachteil ist die starke Hygroskopie von AlQ₃, ebenso wie die niedrige Elektronenbeweglichkeit, was zu höheren Spannungen und damit zu einer niedrigeren Leistungseffizienz führt. Um Kurzschlüsse im Display zu vermeiden, würde man gern die Schichtdicke erhöhen; dies ist mit AlQ₃ wegen der geringen Ladungsträgerbeweglichkeit und der daraus resultierenden Spannungserhöhung nicht möglich. Als sehr ungünstig erweist sich weiterhin die Eigenfarbe von AlQ₃ (im Feststoff gelb), die gerade bei blauen OLEDs durch Reabsorption und schwache Reemission zu Farbverschiebungen führen kann. Hier sind blaue OLEDs nur mit starken Effizienz- bzw. Farborteinbußen darstellbar. Trotz der genannten Nachteile stellt AlQ₃ in OLEDs bislang immer noch den besten Kompromiss für die verschiedenartigen Anforderungen an ein Elektronentransportmaterial in OLEDs dar.

Das Dokument EP 1 589 089 A offenbart eine lichtemittierende Vorrichtung, die Licht durch elektrische Energie emittiert, die eine oder mehrere Schichten aus organischen Dünnschichten aufweist, die zwischen einer Anode und einer Kathode gebildet sind, dadurch gekennzeichnet, dass eine organische Dünnschicht eine Verbindung mit einem anellierten Anthracen-Grundgerüst enthält.

Es besteht also weiterhin Bedarf an verbesserten Materialien, insbesondere Host-Materialien für blau fluoreszierende Emitter und Host-Materialien für Triplett-Emitter, aber auch an Emittern, Lochtransportmaterialien und Elektronentransportmaterialien, die in organischen elektronischen Vorrichtungen zu guten Effizienzen und gleichzeitig zu hohen Lebensdauern führen, die bei der Herstellung und beim Betrieb der Vorrichtung zu reproduzierbaren Ergebnissen führen und die synthetisch einfach zugänglich sind.

Überraschend wurde gefunden, dass Verbindungen, die neue, unten beschriebene Struktureinheiten enthalten, deutliche Verbesserungen gegenüber dem Stand der Technik aufweisen. Mit diesen Materialien ist eine Steigerung der Effizienz und der Lebensdauer der organischen elektronischen Vorrichtung im Vergleich zu Materialien gemäß dem Stand der Technik möglich. Weiterhin sind diese Materialien sehr gut für die Verwendung in organischen elektronischen Vorrichtungen geeignet, da sie eine hohe Glasübergangstemperatur aufweisen.

Gegenstand der Erfindung ist eine organische Elektrolumineszenzvorrichtung, definiert wie in Anspruch 1.

Bevorzugt weist die Verbindung gemäß Formel (2), (3) bzw. (4) eine Glasübergangstemperatur T_{g} von größer als 70 °C auf, besonders bevorzugt größer als 100 °C, ganz besonders bevorzugt größer als 130 °C.

Eine Arylgruppe im Sinne dieser Erfindung enthält 6 bis 40 C-Atome; eine Heteroarylgruppe im Sinne dieser Erfindung enthält 2 bis 40 C-Atome und mindestens 1 Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe im Sinne der unten folgenden Definition verstanden.

Ein aromatisches Ringsystem im Sinne dieser Erfindung enthält 6 bis 40 C-Atome im Ringsystem. Ein heteroaromatisches Ringsystem im Sinne dieser Erfindung enthält 2 bis 40 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroaryl gruppen durch eine kurze, nicht-aromatische Einheit (bevorzugt weniger als 10 % der von H verschiedenen Atome), wie z. B. ein sp³-hybridisiertes C-, N- oder O-Atom, unterbrochen sein können. So sollen beispielsweise auch Systeme wie 9,9'-Spirobifluoren, 9,9-Diarylfluoren, Triarylamin, Diarylether, etc. als aromatische Ringsysteme im Sinne dieser Erfindung verstanden werden. Dabei kann ein Teil des aromatischen oder heteroaromatischen Ringsystems auch eine kondensierte Gruppe im Sinne der folgenden Definition sein.

Unter einer kondensierten Arylgruppe im Sinne dieser Erfindung wird ein Ringsystem mit 10 bis 40 aromatischen Ringatomen verstanden, in dem mindestens zwei aromatische Ringe miteinander "verschmolzen", d. h. durch Anellierung einander ankondensiert sind, also mindestens eine gemeinsame Kante und ein gemeinsames aromatisches π-Elektronensystem aufweisen. Unter einer kondensierten Heteroarylgruppe im Sinne dieser Erfindung wird ein Ringsystem mit 8 bis 40 aromatischen Ringatomen verstanden, in dem mindestens zwei aromatische oder heteroaromatische Ringe, von denen mindestens einer heteroaromatisch ist, einander ankondensiert sind. Diese Ringsysteme können substituiert oder unsubstituiert sein. Beispiele für kondensierte Aryl- oder Heteroarylgruppen sind Naphthalin, Chinolin, Benzothiophen, Anthracen, Phenanthren, Phenanthrolin, Pyren, Perylen, Chrysen, Acridin, etc., während beispielsweise Biphenyl keine kondensierte Arylgruppe darstellt, da dort keine gemeinsame Kante zwischen den beiden Ringsystemen vorliegt. Auch beispielsweise Fluoren stellt kein kondensiertes aromatisches Ringsystem dar, da die beiden Phenyl-Einheiten dort kein gemeinsames aromatisches Ringsystem ausbilden.

Im Rahmen der vorliegenden Erfindung werden unter einer C₁- bis C₄₀-Alkylgruppe, in der auch einzelne H-Atome oder CH₂-Gruppen durch die oben genannten Gruppen substituiert sein können, besonders bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer C₁- bis C₄₀-Alkoxygruppe werden besonders bevorzugt Methoxy, Trifluormethoxy, Ethoxy, n-Propoxy, i-Propoxy, n-Butoxy, i-Butoxy, s-Butoxy, t-Butoxy oder 2-Methylbutoxy verstanden. Unter einem aromatischen oder heteroaromatischen Ringsystem mit 5 - 40 aromatischen Ringatomen, welches noch jeweils mit den oben genannten Resten R substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Phenanthren, Pyren, Chrysen, Perylen, Fluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Ganz besonders bevorzugt sind Strukturen gemäß den Formeln (2a), (3a) oder (4a), 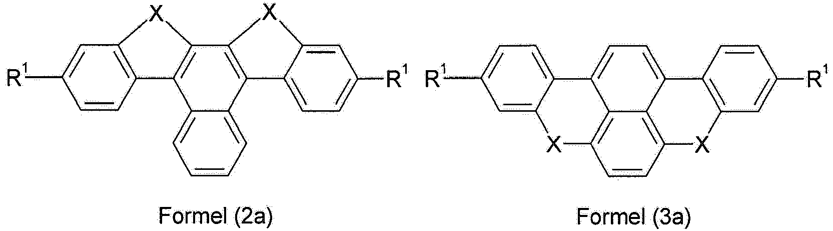 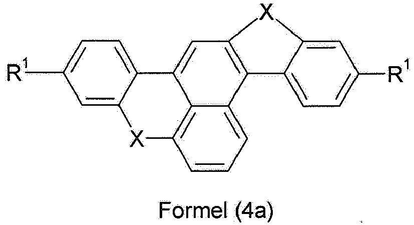 wobei die Symbole X und R¹ dieselbe Bedeutung haben, wie oben beschrieben, und die Naphthalingruppe durch einen oder mehrere Reste R¹ substituiert sein kann.

Besonders bevorzugt sind in den Strukturen gemäß Formel (2a), (3a) und (4a) beide Reste R¹ ungleich Wasserstoff. Weiterhin bevorzugt ist in diesen Strukturen die zentrale Naphthalingruppe unsubstituiert.

Bevorzugt sind weiterhin Verbindungen gemäß Formel (2), (3) und (4) bzw. (2a), (3a) und (4a), in denen das Symbol R¹ gleich oder verschieden bei jedem Auftreten für H, F, C(=O)Ar, P(=O)(Ar)₂, CR²=CR²Ar, eine geradkettige Alkylgruppe mit 1 bis 5 C-Atomen oder verzweigte Alkylgruppe mit 3 bis 5 C-Atomen, wobei eine oder mehrere nicht benachbarte CH₂-Gruppen durch -R²C=CR²-, -C≡C- oder -O- ersetzt sein können und wobei ein oder mehrere H-Atome durch F ersetzt sein können, oder eine Arylgruppe mit 6 bis 16 C-Atomen oder Heteroarylgruppe mit 2 bis 16 C-Atomen oder eine Spirobifluorengruppe, die jeweils mit einem oder mehreren Resten R² substituiert sein kann, oder eine Kombination aus zwei oder drei dieser Systeme steht. Bei Einbau in Polymeren, Oligomeren oder Dendrimeren sind auch lineare oder verzweigte Alkylketten mit bis zu 10 C-Atomen bevorzugt.

Bevorzugt sind weiterhin Verbindungen gemäß Formel (2), (3) und (4) bzw. (2a), (3a) und (4a), in denen das Symbol R¹ gleich oder verschieden bei jedem Auftreten für eine Gruppe N(Ar)₂ der Formel (5) oder (6) steht, 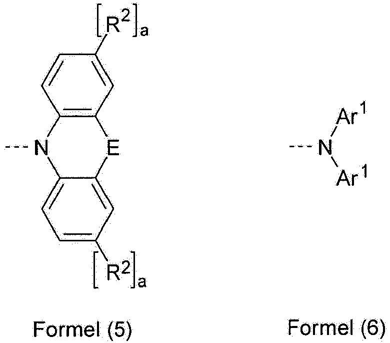 wobei R² die oben aufgeführte Bedeutung hat und weiterhin gilt:
- E: steht für eine Einfachbindung, O, S, N(R²) oder C(R²)₂;
- Ar¹: ist gleich oder verschieden bei jedem Auftreten eine Aryl- oder Heteroarylgruppe mit 5 bis 20 aromatischen Ringatomen oder eine Triarylamingruppe mit 15 bis 30 aromatischen Ringatomen, welche jeweils mit einem oder mehreren Resten R² substituiert sein kann, bevorzugt eine Aryl- oder Heteroarylgruppe mit 6 bis 14 aromatischen Ringatomen oder eine Triarylamingruppe mit 18 bis 22 aromatischen Ringatomen, welche jeweils mit einem oder mehreren Resten R² substituiert sein kann;
- a: ist bei jedem Auftreten gleich oder verschieden 0 oder 1.

Besonders bevorzugt steht Ar¹ gleich oder verschieden für Phenyl, ortho-, meta- oder para-Tolyl, para-Fluorphenyl, 1-Naphthyl, 2-Naphthyl, Triphenylamin, Naphthyldiphenylamin oder Dinaphthylphenylamin.

Bevorzugt sind Verbindungen gemäß Formel (2) und (3) bzw. (2a) und (3a), in denen die Symbole X bei jedem Auftreten gleich oder verschieden ausgewählt sind aus C(R¹)₂, N(R¹), P(R¹) und P(=O)(R¹), ganz besonders bevorzugt C(R¹)₂ oder N(R¹), insbesondere C(R¹)₂.

Weiterhin bevorzugt sind symmetrische und symmetrisch substituierte Verbindungen, also Verbindungen gemäß Formel (2) und (3) bzw. (2a) und (3a), in denen die beiden äußeren, nicht-kondensierten Aryl- bzw. Heteroarylgruppen gleich sind und in denen die Symbole X gleich sind. Weiterhin bevorzugt sind in den Strukturen (2a), (3a) und (4a) die Substituenten R¹ gleich gewählt.

Beispiele für Verbindungen gemäß Formel (2), (3) bzw. (4) sind die im Folgenden abgebildeten Strukturen, wobei nicht anspruchsgemäße Strukturen mit * gekennzeichnet sind.

| | |
|---|---|
| 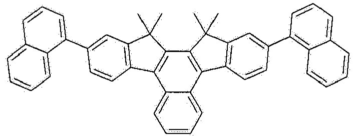 | 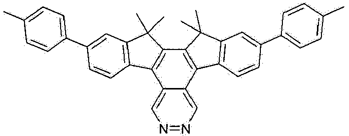 |
| (1) | (2) * |
| 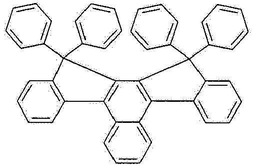 | 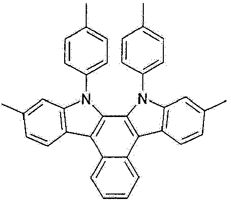 |
| (3) | (4) |
| 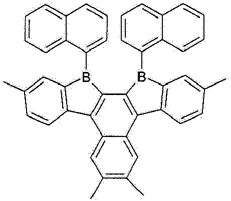 | 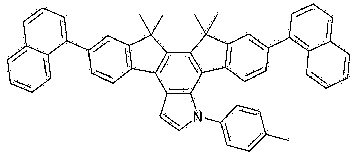 |
| (5) * | (6) |
| 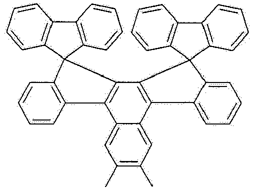 | 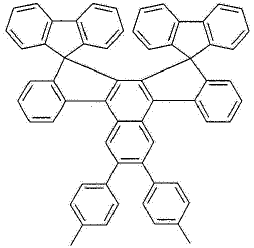 |
| (7) | (8) |
| 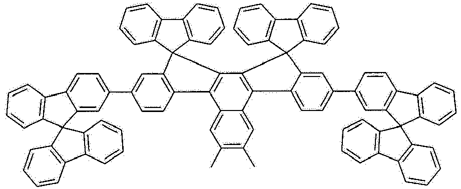 | 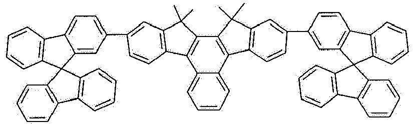 |
| (9) | (10) |
| 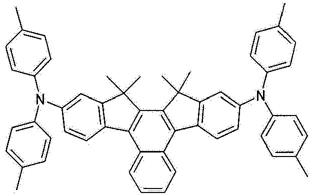 | 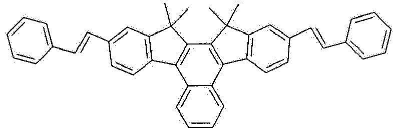 |
| (11) | (12) |
| 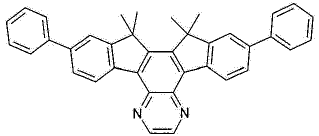 | 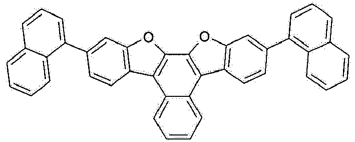 |
| (13) * | (14) |
| 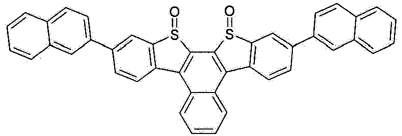 | 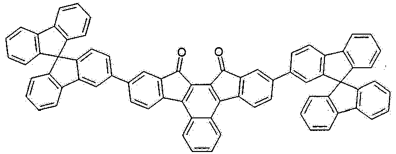 |
| (15) | (16) |
| 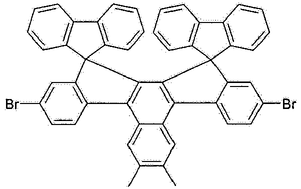 | 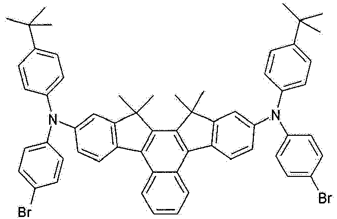 |
| (17) | (18) |
| 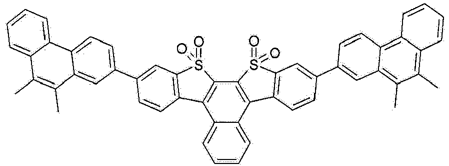 | 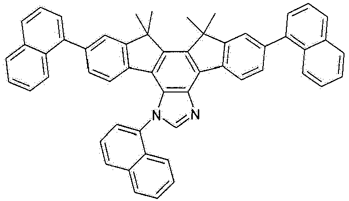 |
| (19) | (20) * |
| 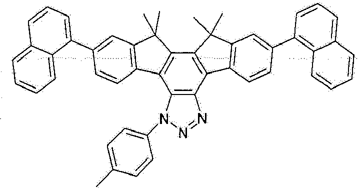 | 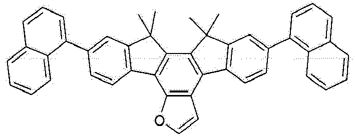 |
| (21) * | (22) * |
| 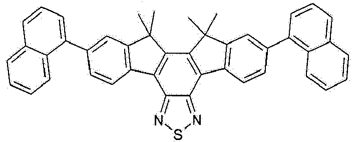 | 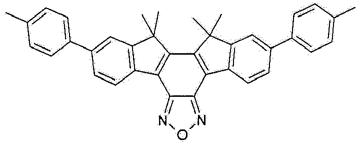 |
| (23) * | (24) * |
| 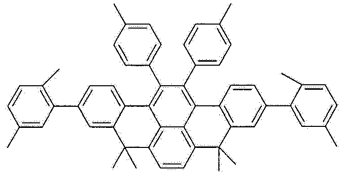 | 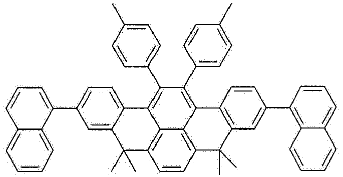 |
| (25) | (26) |
| 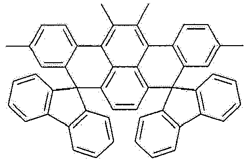 | 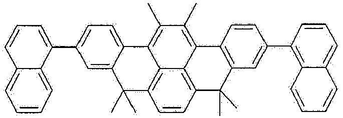 |
| (27) | (28) |
| 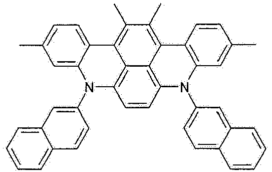 | 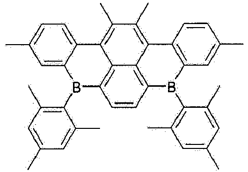 |
| (29) | (30) * |
| 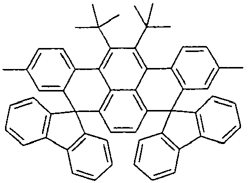 | 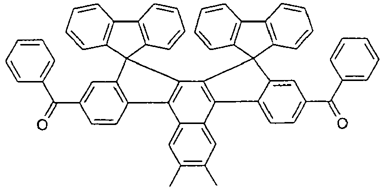 |
| (31) | (32) |
| 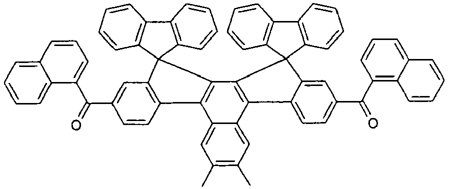 | 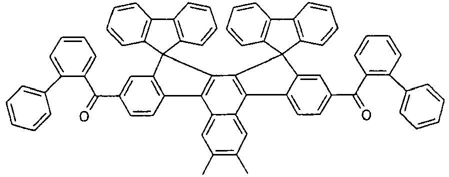 |
| (33) | (34) |
| 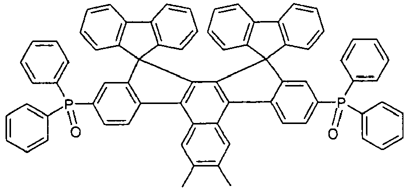 | 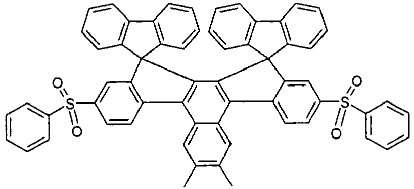 |
| (35) | (36) |
| 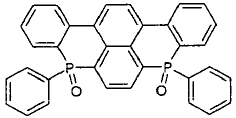 | 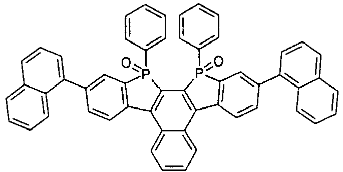 |
| (37) | (38) |
| 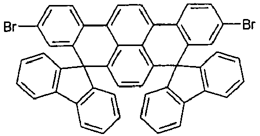 | 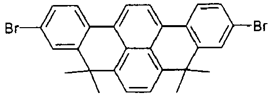 |
| (39) | (40) |
| 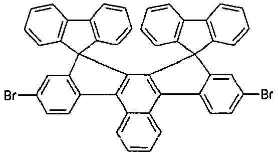 | 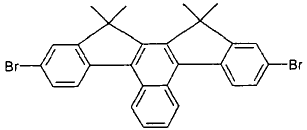 |
| (41) | (42) |
| 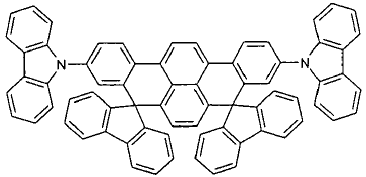 | 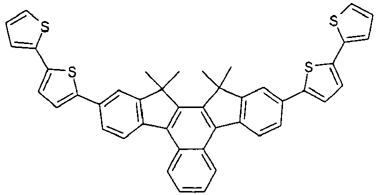 |
| (43) | (44) |
| 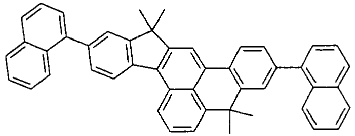 | 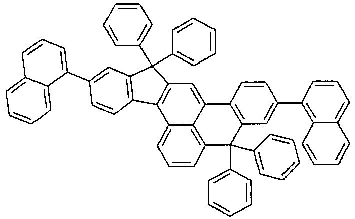 |
| (45) | (46) |
| 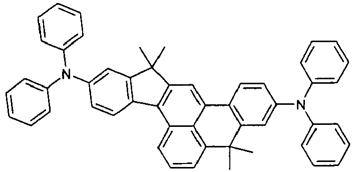 | 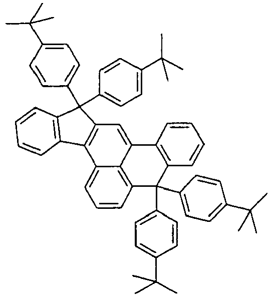 |
| (47) | (48) |
| 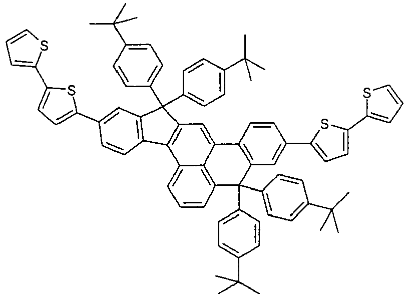 | 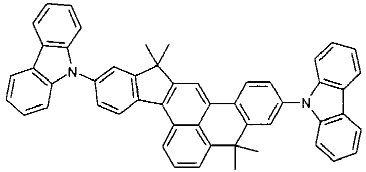 |
| (49) | (50) |
| 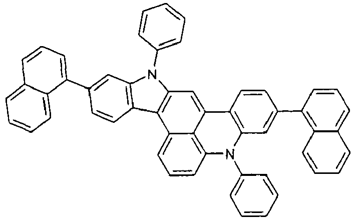 | 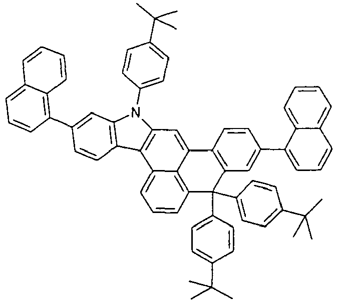 |
| (51) | (52) |
| 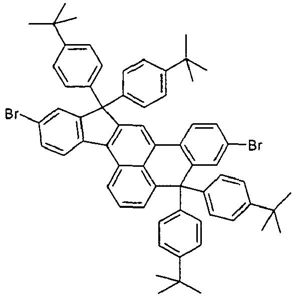 | 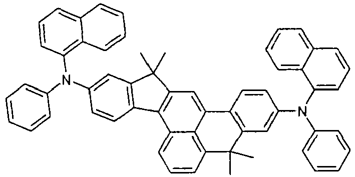 |
| (53) | (54) |
| 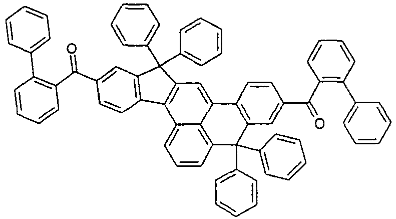 | 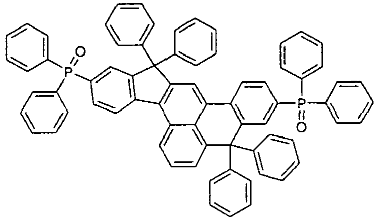 |
| (55) | (56) |

Die oben beschriebenen erfindungsgemäßen Verbindungen, insbesondere Verbindungen, welche mit reaktiven Abgangsgruppen wie Brom, Iod, Boronsäure oder Boronsäureester substituiert sind, z. B. Verbindungen gemäß den Strukturen 17, 18, 39, 40, 41, 42 und 53, können auch als Comonomere zur Erzeugung entsprechender konjugierter, teilkonjugierter oder nicht-konjugierter Polymere, Oligomere oder auch als Kern von Dendrimeren Verwendung finden. Die Polymerisation erfolgt dabei bevorzugt über die Halogenfunktionalität bzw. die Boronsäurefunktionalität.

Die erfindungsgemäßen Verbindungen können nach dem Fachmann bekannten Syntheseschritten, wie z. B. Bromierung, Suzuki-Kupplung, Hartwig-Buchwald-Kupplung, Acylierung, Alkylierung, oxidative Cyclisierung, etc., dargestellt werden.

Verbindungen mit Benzo[rst]pentaphen-5,8-dion-Grundgerüst können beispielsweise durch oxidative Cyclisierung von 1,4-Dibenzoylnaphthalin in einer eutektischen Aluminiumchlorid-Natriumchlorid-Schmelze dargestellt werden (Schema 1).

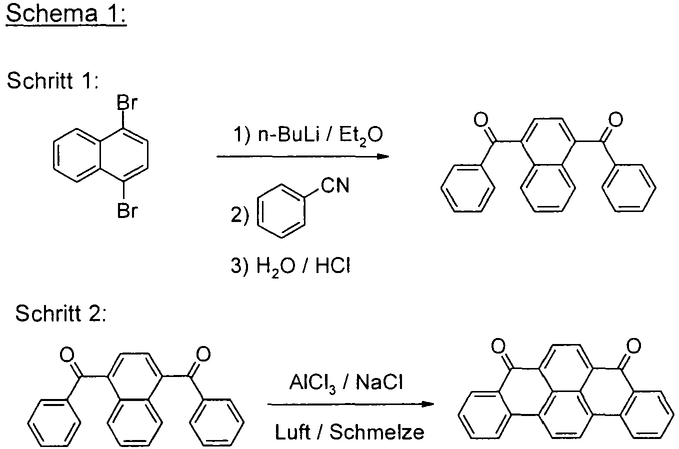

Die anschließende Reduktion des so erhaltenen Benzo[rst]pentaphen-5,8-dion-Grundgerüsts mit Hydrazinhydrat nach Wolf-Kishner bzw. Huang-Minlon, gefolgt von einer Methylierung und einer abschließenden Bromierung, ergibt das Synthon 3,10-Dibrom-5,5,8,8-tetramethylbenzo[rst]pentaphen (Schema 2).

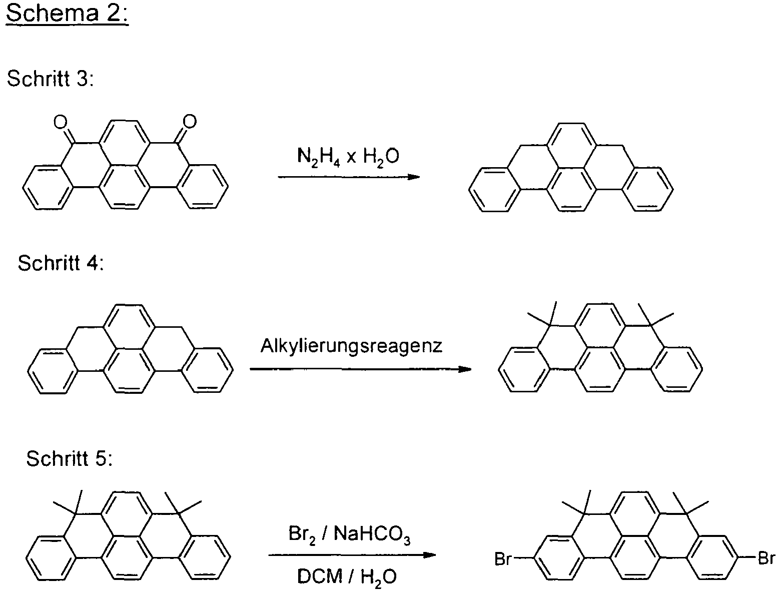

3,10-Dibrom-5,5,8,8-tetramethyl-benzo[rst]pentaphen kann dann z. B. durch Suzuki-Kupplung mit Arylboronsäuren und Arylboronsäurederivaten zu erweiterten aromatischen Kohlenwasserstoffen, durch Buchwald-Kupplung mit Diarylaminen zu Triarylamin-Derivaten oder via Lithiierung und Reaktion mit Elektrophilen wie Benzonitril und anschließender saurer Hydrolyse zu Ketonen oder mit Chlordiphenylphosphinen und anschließender Oxidation zu Phosphinoxiden umgesetzt werden (Schema 3).

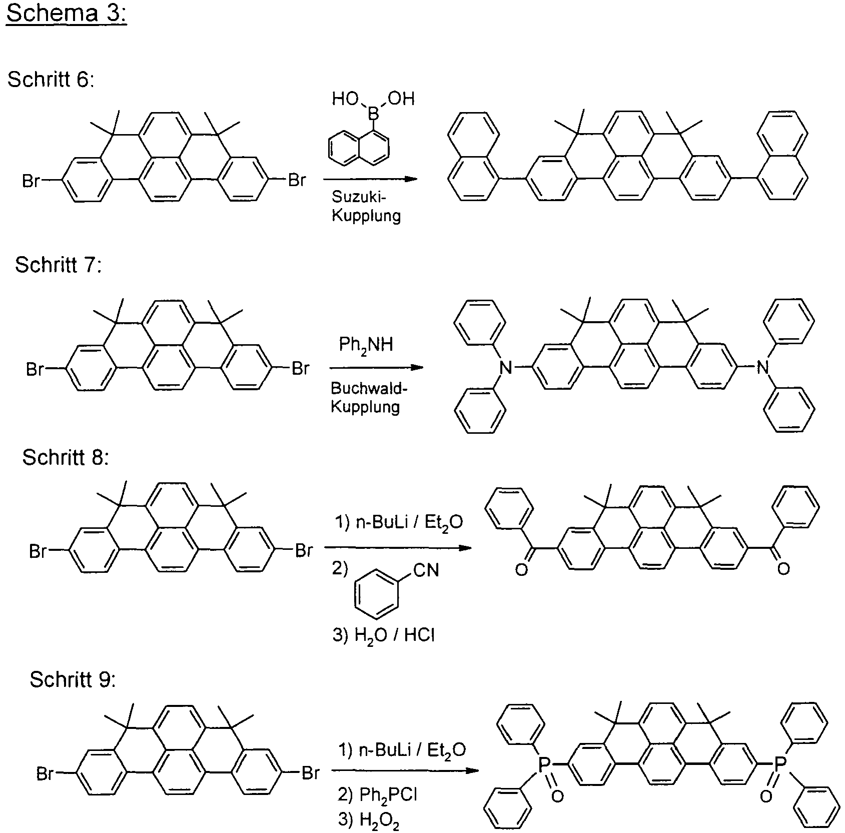

Selbstverständlich kann die Bromierung auch am Benzo[rst]pentaphen-5,8-dion-Grundgerüst erfolgen. Eine anschließende Umsetzung mit Biphenyl-2-magnesiumbromid und eine säurekatalysierte Cyclisierung des intermediären Triarylmethanols ergibt die entsprechende Spiro-Verbindung des Benzo[rst]pentaphens (Schema 4), welche wie in Schema 3 beschrieben weiter funktionalisiert werden kann.

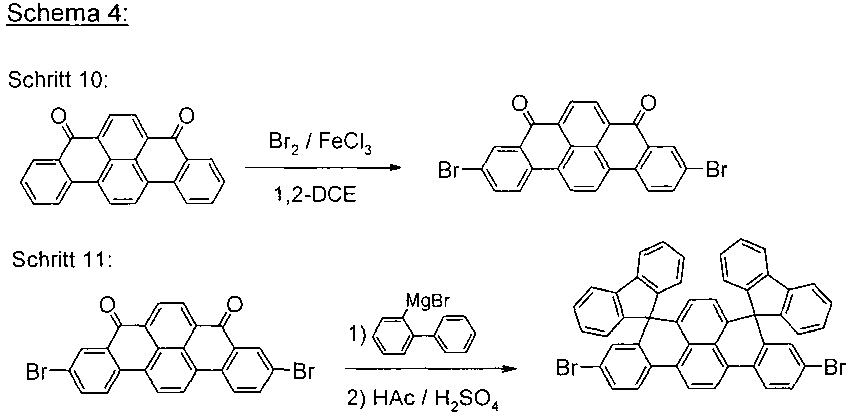

Benz[c]indeno[2,1-a]fluoren-13,14-dion kann gemäß Bulletin Chem. Soc. Jpn. 1977, 50(1), 273 dargestellt werden. Die Bromierung ist mit elementarem Brom in Gegenwart von wasserfreiem Eisen(III)chlorid möglich (analog Schema 2). Das so gewonnene 2,11-Dibrombenz[c]-indeno[2,1-a]fluoren-13,14-dion kann analog den Schemata 2, 3 und 4 weiter funktionalisiert werden.

Ein alternativer Aufbau der Grundgerüste ist ausgehend von 1,4-Bis(2-methoxycarbonylphenyl)naphthalin möglich, wie in Schema 5 dargestellt. Dieses wird mit einem Organometallreagenz zum entsprechenden tertiären Alkohol umgesetzt, der säurekatalysiert cyclisiert wird. Das Verhältnis der gebildeten Isomere hängt dabei von den Substituenten und den genauen Synthesebedingungen ab. Die weitere Funktionalisierung kann wie oben beschrieben erfolgen.

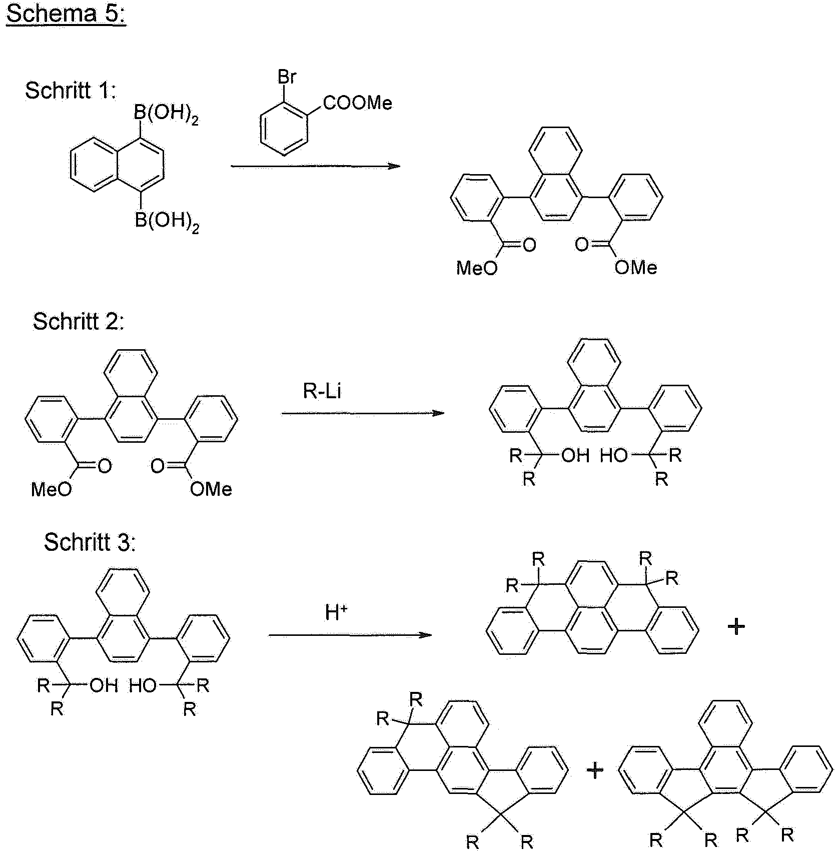

Weiterhin können die bromierten Verbindungen entweder direkt oder nach Überführung in ein Boronsäurederivat als Monomere für Polymere, Oligomere oder Dendrimere eingesetzt werden.

Bei der Synthese können, je nach Synthesebedingungen, sowohl die 5-Ring/5-Ring-Derivate wie auch die 6-Ring/6-Ring-Derivate, die 5-Ring/6-Ring-Derivate oder Mischungen dieser Verbindungen entstehen. Diese können entweder getrennt und als Reinverbindungen weiterverarbeitet werden, oder sie können auch als Mischung eingesetzt werden.

Die Verbindungen gemäß Formel (2), (3) oder (4) sind sehr gut für den Einsatz in organischen Elektrolumineszenzvorrichtungen (OLEDs, PLEDs) geeignet.

Abhängig von der Substitution wird die Verbindung in unterschiedlichen Funktionen in der OLED eingesetzt.

Außer Kathode, Anode und der emittierenden Schicht kann die organische Elektrolumineszenzvorrichtung noch weitere Schichten enthalten. Diese können beispielsweise sein: Lochinjektionsschicht, Lochtransportschicht, Elektronentransportschicht, Elektroneninjektionsschicht und/oder Charge-Generation Layer (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer). Es sei aber darauf hingewiesen, dass nicht notwendigerweise jede dieser Schichten vorhanden sein muss.

In einer weiteren bevorzugten Ausführungsform der Erfindung enthält die organische Elektrolumineszenzvorrichtung mehrere emittierende Schichten, wobei mindestens eine Schicht mindestens eine Verbindung gemäß Formel (2), (3) oder (4) enthält. Besonders bevorzugt weisen diese Emissionsschichten insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die gelbes, orange oder rotes Licht emittieren. Insbesondere bevorzugt sind Dreischichtsysteme, wobei mindestens eine dieser Schichten mindestens (4) enthält und wobei die drei Schichten blaue, grüne und orange oder rote Emission zeigen (für den prinzipiellen Aufbau siehe z. B. WO 05/011013). Ebenso eignen sich für weiße Emission Emitter, welche breitbandige Emissionsbanden aufweisen und dadurch weiße Emission zeigen.

Bei Verwendung der Verbindungen gemäß Formel (2), (3) oder (4) als Host für einen fluoreszierenden Dotanden sind bevorzugt ein oder mehrere Substituenten R¹ gewählt aus einfachen oder kondensierten Aryl- oder Heteroarylgruppen, insbesondere Phenyl, ortho-, meta- oder para-Biphenyl, 1- oder 2-Naphthyl, Anthryl, insbesondere Phenylanthryl oder 1- oder 2-Naphthylanthryl, 2-Fluorenyl und 2-Spirobifluorenyl, welche jeweils durch einen oder mehrere Reste R² substituiert sein können. Dies gilt insbesondere für die Reste R¹ an den Strukturen gemäß Formel (2a), (3a) und (4a).

Unter einem Hostmaterial wird in einem System aus Host und Dotand diejenige Komponente verstanden, die in dem System im höheren Anteil vorliegt. Bei einem System aus einem Host und mehreren Dotanden wird als Host diejenige Komponente verstanden, deren Anteil der höchste in der Mischung ist.

Der Anteil des Hostmaterials gemäß der Formel (2), (3) oder (4) in der emittierenden Schicht beträgt zwischen 50.0 und 99.9 Gew.-%, bevorzugt zwischen 80.0 und 99.5 Gew.-%, besonders bevorzugt zwischen 90.0 und 99.0 Gew.-%. Entsprechend beträgt der Anteil des Dotanden zwischen 0.1 und 50.0 Gew.-%, bevorzugt zwischen 0.5 und 20.0 Gew.-%, besonders bevorzugt zwischen 1.0 und 10.0 Gew.-%.

In fluoreszierenden Vorrichtungen ist der Dotand bevorzugt ausgewählt aus der Klasse der Monostyrylamine, der Distyrylamine, der Tristyrylamine, der Tetrastyrylamine und der Arylamine. Unter einem Monostyrylamin wird eine Verbindung verstanden, die eine Styrylgruppe und mindestens ein Amin enthält, welches bevorzugt aromatisch ist. Unter einem Distyrylamin wird eine Verbindung verstanden, die zwei Styrylgruppen und mindestens ein Amin enthält, welches bevorzugt aromatisch ist. Unter einem Tristyrylamin wird eine Verbindung verstanden, die drei Styrylgruppen und mindestens ein Amin enthält, welches bevorzugt aromatisch ist. Unter einem Tetrastyrylamin wird eine Verbindung verstanden, die vier Styrylgruppen und mindestens ein Amin enthält, welches bevorzugt aromatisch ist. Unter einem Arylamin bzw. einem aromatischen Amin im Sinne dieser Erfindung wird eine Verbindung verstanden, die drei aromatische oder heteroaromatische Ringsysteme direkt an den Stickstoff gebunden enthält, davon bevorzugt mindestens ein kondensiertes Ringsystem mit mindestens 14 aromatischen Ringatomen. Die Styrylgruppen sind besonders bevorzugt Stilbene, die auch an der Doppelbindung oder an den Aromaten weiter substituiert sein können. Beispiele für derartige Dotanden sind substituierte oder unsubstituierte Tristilbenamine oder weitere Dotanden, die beispielsweise in WO 06/000388 und in den nicht offen gelegten Patentanmeldungen DE 102004031000.9, EP 04028407.7 und EP 05001891.0 beschrieben sind. Außerdem sind Verbindungen gemäß DE 102005023437.2 bevorzugt.

Bei Verwendung der Verbindungen gemäß Formel (2), (3) oder (4) als Host für phosphoreszierende Dotanden enthalten bevorzugt ein oder mehrere Substituenten R¹ mindestens eine Gruppe C=O, P(=O)(R²) und/oder SO₂. Besonders bevorzugt sind diese Gruppen direkt an die erfindungsgemäße zentrale Einheit gebunden und enthalten weiterhin besonders bevorzugt noch einen bzw. im Fall des Phosphinoxids zwei weitere aromatische Substituenten. Dies gilt insbesondere für die Reste R¹ an den Strukturen gemäß Formel (2a), (3a) und (4a).

In phosphoreszierenden Vorrichtungen ist der Dotand bevorzugt ausgewählt aus der Klasse der Metallkomplexe, enthaltend mindestens ein Element der Ordnungszahl größer 20, bevorzugt größer 38 und kleiner 84, besonders bevorzugt größer 56 und kleiner 80. Bevorzugt werden als Phosphoreszenzemitter Metallkomplexe verwendet, die Molybdän, Wolfram, Rhenium, Ruthenium, Osmium, Rhodium, Iridium, Palladium, Platin, Silber, Gold oder Europium enthalten, insbesondere Iridium oder Platin. Generell eignen sich hierfür phosphoreszierende Materialien, wie sie gemäß dem Stand der Technik verwendet werden.

Als Hostmaterialien kommen dann verschiedene Stoffklassen in Frage. Bevorzugte Hostmaterialien sind ausgewählt aus den Klassen der Oligo-arylene (z. B. 2,2',7,7'-tetraphenyl-spirobifluoren gemäß EP 676461 oder Dinaphthylanthracen), insbesondere der Oligo-arylene enthaltend kondensierte aromatische Gruppen, der Oligo-arylenvinylene (z. B. DPVBi oder Spiro-DPVBi gemäß EP 676461), der polypodalen Metallkomplexe (z. B. gemäß WO 04/081017), der lochleitenden Verbindungen (z. B. gemäß WO 04/058911), der elektronenleitenden Verbindungen, insbesondere Ketone, Phosphinoxide, Sulfoxide, etc. (z. B. gemäß WO 05/084081 und WO 05/084082), der Atropisomere (z. B. gemäß der nicht offen gelegten Anmeldung EP 04026402.0) oder der Boronsäurederivate (z. B. gemäß der nicht offen gelegten Anmeldung EP 05009643.7). Weiterhin kommen als Hostmaterialien die oben beschriebenen erfindungsgemäßen Verbindungen in Frage. Besonders bevorzugte Hostmaterialien sind außer den erfindungsgemäßen Verbindungen ausgewählt aus den Klassen der Oligoarylene, enthaltend Naphthalin, Anthracen und/oder Pyren oder Atropisomeren dieser Verbindungen, der Oligo-arylenvinylene, der Ketone, der Phosphinoxide und der Sulfoxide. Ganz besonders bevorzugte Hostmaterialien sind außer den erfindungsgemäßen Verbindungen ausgewählt aus den Klassen der Oligoarylene, enthaltend Anthracen und/oder Pyren oder Atropisomeren dieser Verbindungen, der Phosphinoxide und der Sulfoxide. Unter einem Oligoarylen im Sinne dieser Erfindung soll eine Verbindung verstanden werden, in der mindestens drei Aryl- bzw. Arylengruppen aneinander gebunden sind.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Druck kleiner 10⁻⁵ mbar, bevorzugt kleiner 10⁻⁶ mbar, besonders bevorzugt kleiner 10⁻⁷ mbar aufgedampft.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht.

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (2), (3) bzw. (4) nötig. Hohe Löslichkeit lässt sich durch geeignete Substitution der Verbindungen erreichen.

Die erfindungsgemäßen Verbindungen weisen bei Verwendung in organischen Elektrolumineszenzvorrichtungen eine hohe Effizienz und eine hohe Stabilität, was sich vor allem in einer hohen Lebensdauer zeigt, auf. Außerdem weisen die Verbindungen eine hohe Glasübergangstemperatur auf.

Die Erfindung wird durch die nachfolgenden Beispiele näher erläutert, ohne sie dadurch einschränken zu wollen.

### Beispiele:

Die nachfolgenden Synthesen wurden, sofern nicht anders angegeben, unter einer Schutzgasatmosphäre durchgeführt. Die Edukte wurden von den Firmen ALDRICH bzw. ABCR bezogen. 1,4-Naphthalindiboronsäure wurde gemäß Journal of Organic Chemistry 2000, 65(13), 3952-3960 synthetisiert.

### Beispiel 1: Synthese von 4,10-Tetramethyl-4H-10H-fluoreno[4,3,2-de]anthracen und 5,8-Tetramethyl-5,8-dihydro-benzo[rst]pentaphen

### a) 1,4-Bis(2-methoxycarbonylphenyl)naphthalin

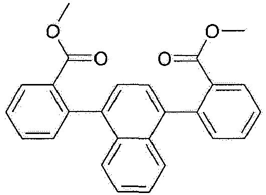

Eine gut gerührte, entgaste Suspension aus 21.6 g (7.1 mmol) 2-Brombenzoesäuremethylester, 10.1 g (28 mmol) 1,4-Naphthalindiboronsäure und 18.9 g (6.6 mmol) Triskaliumphosphat in einem Gemisch aus 350 ml Wasser und 350 ml THF wird mit 1.55 g (0.1 mmol) Pd(PPh₃)₄ versetzt und 60 h unter Rückfluss erhitzt. Nach Erkalten wird die organische Phase abgetrennt, dreimal mit 200 ml Wasser und einmal mit 200 ml gesättigter Kochsalzlösung gewaschen und anschließend über Magnesiumsulfat getrocknet. Die organische Phase wird im Vakuum zur Trockene einrotiert. Der so erhaltene graue Rückstand wird aus Dioxan umkristallisiert. Die ausgefallenen Kristalle werden abgesaugt, mit 50 ml Ethanol gewaschen und anschließend im Vakuum getrocknet. Ausbeute: 13 g, 82 % d. Th.

### b) 1,4-Bis(2-methanol-α,α-dimethyl)phenyl)naphthalin

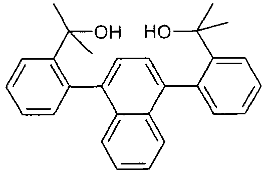

30 g (75 mmol) 1,4-Bis(2-methoxycarbonylphenyl)naphthalin werden in 2000 ml THF unter Stickstoff vorgelegt, auf -78 °C gekühlt und tropfenweise mit 175 ml (378 mmol) 2.2 M Methyllithium-Lösung versetzt. Anschließend wird 16 h bei -78°C gerührt. Man lässt über Nacht auf Raumtemperatur kommen. Nach Hydrolyse mit 125 ml gesättigter NH₄Cl-Lösung wird der Niederschlag abgesaugt und mit Ethylacetat gewaschen. Das Filtrat wird zweimal mit Wasser extrahiert und die organische Phase über Na₂SO₄ getrocknet. Der Rückstand nach Abdampfen des Lösemittels wird chromatographisch gereinigt (Toluol /Ethylacetat 6:4). Ausbeute: 27 g, 93 % d. Th.

### c) 4,10-Tetramethyl-4H-10H-fluoreno[4,3,2-de]anthracen und 5,8-Tetramethyl-5,8-dihydro-benzo[rst]pentaphen

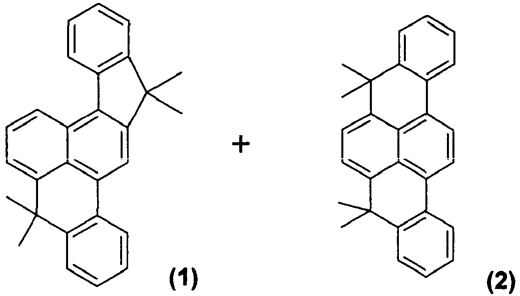

200 g Polyphosphorsäure werden vorgelegt. Anschließend werden 25 g (63.05 mmol) 1,4-Bis(2-methanol-α,α-dimethyl)phenyl)naphthalin zugegeben. Die Mischung wird 20 h bei Raumtemperatur gerührt. Danach wird die Mischung langsam mit Eiswasser versetzt. Der ausgefallene Feststoff wird abgesaugt, im Trockenschrank getrocknet und aus Ethylacetat umkristallisiert.

### Ausbeute:

4,10-Tetramethyl-4H-10H-fluoreno[4,3,2-de]anthracen **(1):** 9.9 g (45 % d. Th.)
5,8-Tetramethyl-5,8-dihydro-benzo[rst]pentaphen **(2):** 6.6 g (30 % d. Th.)

### Beispiel 2: Synthese von 5,8-Tetra(p-tert-butylphenyl)-5,8-dihydro-benzo[rst]pentaphen und 4,10-Tetra(p-tert-butylphenyl)-4H-10H-fluoreno[4,3,2-de]anthracen

### a) 1,4-Bis[2-methanol-α,α-di(p-tert-butylphenyl)phenyl]naphthalin

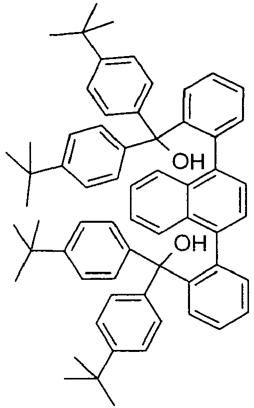

8.5 g (40 mmol) 1-Brom-4-tert-butylbenzol werden in 50 ml THF unter Stickstoff vorgelegt, auf -75 °C gekühlt, tropfenweise mit 25 ml (40 mmol) 1.6 M n-Butyllithium-Lösung versetzt und 2 h bei dieser Temperatur gerührt. Anschließend werden 3.56 g (9 mmol) 1,4-Bis(2-methoxycarbonyl-phenyl)naphthalin (synthetisiert gemäß Beispiel 1a), gelöst in 50 ml THF, so zugetropft, dass die Temperatur -65 °C nicht übersteigt. Man lässt über Nacht auf Raumtemperatur kommen. Nach Hydrolyse mit 200 ml Wasser wird der Niederschlag abgesaugt, mit EtOH nachgewaschen und aus Ethylacetat umkristallisiert. Ausbeute: 15.8 g, 85 % d. Th.

### b) 5,8-Tetra(p-tert-butylphenyl)-5,8-dihydro-benzo[rst]pentaphen und 4,10-Tetra(p-tert-butylphenyl)-4H-10H-fluoreno[4,3,2-de]anthracen

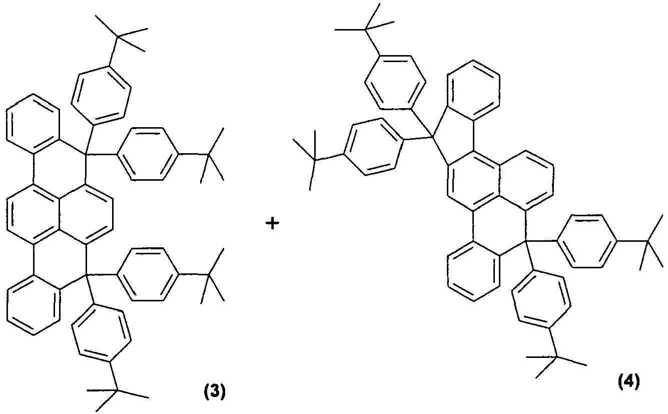

9.7 g (11.2 mmol) 1,4-Bis[2-methanol-α,α-di(p-*tert*-butylphenyl)phenyl]-naphthalin werden in 120 ml Eisessig vorgelegt und 10 min. gerührt. Anschließend werden 0.5 ml HCl (konz.) zugegeben und 1 h unter Rückfluss gekocht. Danach wird die Mischung langsam mit Eiswasser versetzt. Der ausgefallene Feststoff wird abgesaugt, im Trockenschrank getrocknet und aus Toluol umkristallisiert.

### Ausbeute:

5,8-Tetra(p-*tert*-butylphenyl)-5,8-dihydro-benzo[rst]pentaphen **(3):** 5.8 g, 50 % d. Th.
4,10-Tetra(p-*tert*-butylphenyl)-4H-10H-fluoreno[4,3,2-de]anthracen **(4):** 2.8 g, 30 % d. Th.

### Beispiel 3: Synthese von Amin D1

### a) Bromierung von Verbindung (1)

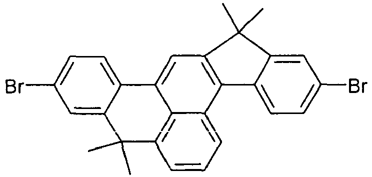

9.0 g (25 mmol) 4,10-Tetramethyl-4H-10H-fluoreno[4,3,2-de]anthracen **(1)** werden unter Lichtausschluss in 300 ml Dichlormethan vorgelegt und auf 5 °C abgekühlt. Es werden 2.7 ml (50 mmol) Brom in 25 ml Dichlormethan innerhalb 15 min. zugetropft und bei 5 °C weitere 7 h gerührt. Nach vollständigem Umsatz wird die Reaktion durch Zugabe von 15 ml Ethanol gestoppt, abgesaugt, mehrfach mit Ethanol gewaschen und anschließend zweimal aus NMP umkristallisiert. Man erhält 11.2 g (86 % d. Th.) eines hellgelben Feststoffs, der gemäß HPLC eine Reinheit von > 99.7 % aufweist.

### b) Synthese von Amin D1

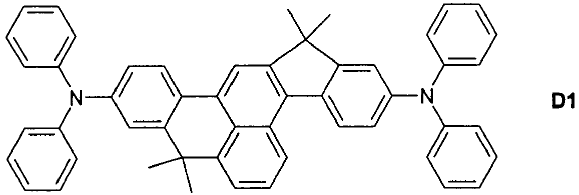

11.2 g (22 mmol) der Bromverbindung aus Beispiel 3a) und 9.7 g (58 mmol) Diphenylamin werden in 250 ml wasserfreiem Toluol suspendiert. Anschließend werden 190 mg (0.9 mmol) Tri-tert-butylphosphin sowie 107 mg (0.5 mmol) Pd(OAc)₂ und 6.3 g (66 mmol) NaO^{t}Bu zugegeben, und das Reaktionsgemisch wird 4 h unter Rückfluss erhitzt. Nach beendeter Reaktion werden 150 ml Wasser zugegeben, der Feststoff abgesaugt, mit Ethanol gewaschen und getrocknet. Nach fünfacher Umkristallisation aus NMP, zweimaligem Auskochen mit Ethanol und anschließender zweifacher Sublimation (330 °C, 2 x 10⁻⁵ mbar) erhält man 11.8 g (77 %) eines hellgelben Feststoffs mit einer Reinheit von > 99.9 % gemäß HPLC.

### Beispiele 4 bis 6: Synthese der Amine D2, D3 und D4

In Analogie zu Beispiel 3 werden aus den Verbindungen **(2), (3)** und **(4)** (synthetisiert gemäß den Beispielen 1 und 2) durch Bromierung und Hartwig-Buchwald-Kupplung die entsprechenden Bis(diphenylamin)-Derivate synthetisiert. Die Strukturen von **D2**, **D3** und **D4** sind im Folgenden abgebildet:

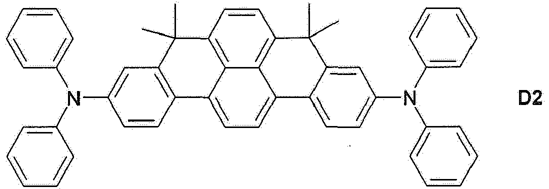

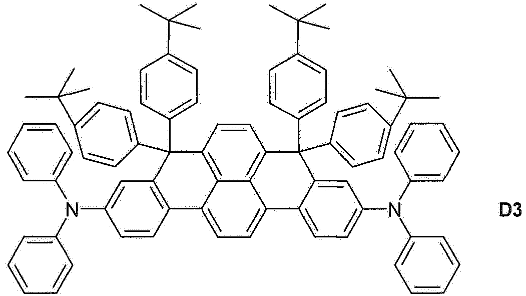

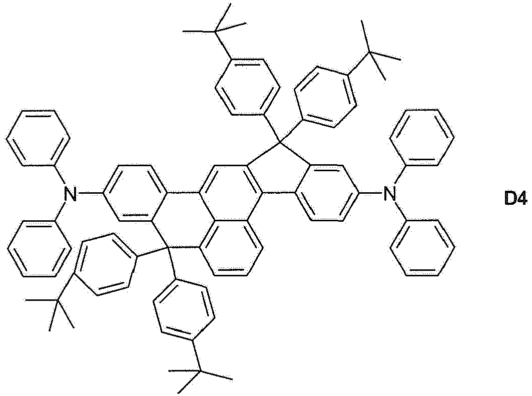

### Beispiel 7: Herstellung der OLEDs. Nicht anspruchsgemäße OLED-Beispiele sind mit # gekennzeichnet.

Die Herstellung der OLEDs erfolgt nach einem allgemeinen Verfahren gemäß WO 04/058911, das im Einzelfall auf die jeweiligen Gegebenheiten (z. B. Schichtdickenvariation, um optimale Effizienz bzw. Farbe zu erreichen) angepasst wird.

In den folgenden Beispielen werden die Ergebnisse verschiedener OLEDs vorgestellt. Der grundlegende Aufbau, die verwendeten Materialien und Schichtdicken, außer der emittierenden Schicht und der Lochinjektionsschicht, sind zur besseren Vergleichbarkeit identisch. Analog dem o. g. allgemeinen Verfahren werden OLEDs mit folgendem Aufbau erzeugt:

| | |
|---|---|
| Lochinjektionsschicht (HIL) | 0 nm oder 20 nm **HIL1,** siehe Tabelle 1 |
| Lochtransportschicht (HTM) | 40 nm oder 20 nm NPB (aufgedampft; N-Naphthyl-N-phenyl-4,4'-diaminobiphenyl) |
| Emissionschicht (EML) | siehe Tabelle 1 für Materialien, Konzentration und Schichtdicke |
| Elektronenleiter (ETL) | 20 nm Alq₃ (bezogen von SynTec; Tris(chinolinato)aluminium(III)) |
| LiF-Al (Kathode) | 1 nm LiF, darauf 150 nm Al. |

Diese OLEDs werden standardmäßig charakterisiert; hierfür werden die Elektrolumineszenzspektren, die Effizienz (gemessen in cd/A), die Leistungseffizienz (gemessen in lm/W) in Abhängigkeit der Helligkeit, berechnet aus Strom-Spannungs-Leuchtdichte-Kennlinien (IUL-Kennlinien), und die Lebensdauer bestimmt. Als Lebensdauer wird die Zeit definiert, nach der die Anfangshelligkeit von 1000 cd/m²auf die Hälfte gesunken ist.

In Tabelle 1 sind die Ergebnisse einiger OLEDs (Beispiele 8 bis 11) zusammengefasst, wobei jeweils die Zusammensetzung der EML und der HTL inklusive der Schichtdicken mit aufgeführt ist. (Beispiele 10 und 11 sind Referenzbeispiele.) Dabei enthalten die OLEDs die erfindungsgemäßen Verbindungen entweder als Lochinjektionsmaterial (Verwendung von **D3** in Beispiel 9 und 11), als Hostmaterial in der emittierenden Schicht (Verwendung von **(3)** in Beispiel 8 und 9) und/oder als Dotand in der emittierenden Schicht (Verwendung von **D3** in Beispiel 10 und 11). Die Strukturen des Hostmaterials **H1** und des Dotanden **D5** sind im Folgenden dargestellt:

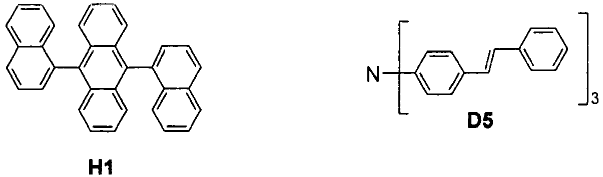

**Tabelle 1**

| Bsp. | HIL | HTL2 | EML | Max. Eff. (cd/A) | U (V) bei 1000 cd/m² | CIE ^{a} | Lebensdauer (h) ^{b} |
|---|---|---|---|---|---|---|---|
| 8 | - | **NPB** | **(3):D5 (5%)** | 3.8 | 6.8 | x=0.15 | 700 |
| | | (40 nm) | (30 nm) | | | y=0.11 | |
| 9 | **D3** | **NPB** | **(3):D5 (5%)** | 4.0 | 6.0 | x=0.15 | 2200 |
| | (20 nm) | (20 nm) | (30 nm) | | | y=0.11 | |
| 10 | - | **NPB** | **H1:D3 (5%)** | 9.0 | 5.6 | x=0.18 | 5500 |
| # | | (40 nm) | (30 nm) | | | y=0.24 | |
| 11 | **D3** | **NPB** | **H1:D3 (5%)** | 11.0 | 5.8 | x=0.18 | 8000 |
| # | (20 nm) | (20 nm) | (30 nm) | | | y=0.24 | |

| | | | | | | | |
|---|---|---|---|---|---|---|---|
| ^{a} CIE-Koordinaten: Farbkoordinaten der Commision Internationale de l'Eclairage 1931. ^{b} Lebensdauer: Zeit bis zum Abfall der Helligkeit auf 50 % der Anfangshelligkeit, gemessen bei einer Anfangshelligkeit von 1000 cd/m². | | | | | | | |

Zusammenfassend kann gesagt werden, dass OLEDs, enthaltend Verbindungen gemäß Formel (1), sehr gute Lebensdauern und Effizienzen aufweisen, wie man leicht Tabelle 1 entnehmen kann. Daher eignen sich diese Verbindungen sehr gut für die Verwendung in OLEDs.

## Patentansprüche

1. Organische Elektrolumineszenzvorrichtung, enthaltend Anode, Kathode und mindestens eine emittierende Schicht, **dadurch gekennzeichnet, dass** mindestens eine Verbindung gemäß Formel (2), (3) oder (4) in einer emittierenden Schicht als Mischung mit mindestens einem fluoreszierenden oder phosphoreszierenden Dotanden eingesetzt wird, 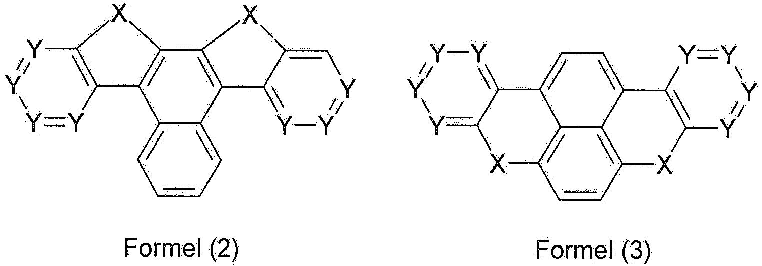 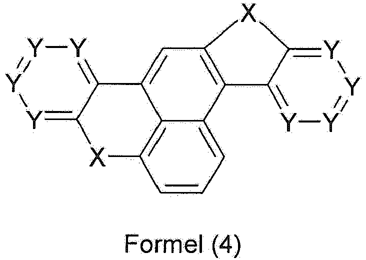 wobei für die verwendeten Symbole und Indizes gilt:
Y ist bei jedem Auftreten gleich oder verschieden gewählt aus CH und CR¹, wobei höchstens zwei Gruppen R¹ je aromatischem Sechsring vorhanden sein dürfen;
R¹ ist bei jedem Auftreten gleich oder verschieden H, F, Cl, Br, I, N(Ar)₂, C(=O)Ar, P(=O)(Ar)₂, S(=O)Ar, S(=O)₂Ar, CR²=CR²Ar, CN, NO₂, Si(R²)₃, B(OR²)₂, eine geradkettige Alkyl-, Alkoxy- oder Thioalkoxygruppe mit 1 bis 40 C-Atomen oder eine verzweigte oder cyclische Alkyl-, Alkoxy- oder Thioalkoxy-gruppe mit 3 bis 40 C-Atomen, die jeweils mit einem oder mehreren Resten R² substituiert sein kann, wobei eine oder mehreren Resten R² substituiert sein kann, wobei eine oder mehrere nicht benachbarte CH₂-Gruppen durch -R²C=CR²-, -C≡C-, Si(R²)₂, Ge(R²)₂, Sn(R²)₂, C=O, C=S, C=Se, C=NR², P(=O)(R²), SO, SO₂, -NR²-, -O-, -S- oder -CONR²- ersetzt sein können und wobei ein oder mehrere H-Atome durch F, Cl, Br, I, CN oder NO₂ ersetzt sein können, oder ein aromatisches oder heteroaromatisches Ringsystem mit 5 bis 40 aromatischen Ringatomen, das jeweils durch einen oder mehrere Reste R² substituiert sein kann, oder eine Aryloxy- oder Heteroaryloxygruppe mit 5 bis 40 aromatischen Ringatomen, die durch einen oder mehrere Reste R² substituiert sein kann, oder eine Kombination dieser Systeme;
Ar ist bei jedem Auftreten ein aromatisches oder heteroaromatisches Ringsystem mit 5-30 aromatischen Ringatomen, das mit einem oder mehreren nicht-aromatischen Resten R¹ substituiert sein kann; dabei können auch zwei Reste Ar am selben Stickstoffatom durch eine Einfachbindung oder eine Brücke X miteinander verknüpft sein;
R² ist bei jedem Auftreten gleich oder verschieden H oder ein aliphatischer, aromatischer oder heteroaromatischer Kohlenwasserstoffrest mit 1 bis 20 C-Atomen; dabei können zwei oder mehrere benachbarte Substituenten R² auch miteinander ein mono- oder polycyclisches aliphatisches oder aromatisches Ringsystem bilden;
X ist bei jedem Auftreten gleich oder verschieden eine bivalente Brücke, ausgewählt aus C(R¹)₂, C=O, C=NR¹, O, S, S=O, SO₂, N(R¹), P(R¹), P(=O)R¹, C(R¹)₂-C(R¹)₂, C(R¹)₂-C(R¹)₂-C(R¹)₂, C(R¹)₂-O und C(R¹)₂-O-C(R¹)₂;
wobei die Naphthalingruppe durch einen oder mehrere Reste R¹ substituiert sein kann,
**dadurch gekennzeichnet, dass** mindestens ein Substituent R¹ vorhanden ist, der mindestens eine Aryl- oder Heteroarylgruppe enthält.

2. Organische Elektrolumineszenzvorrichtung gemäß Anspruch 1, **dadurch gekennzeichnet, dass** die Verbindung der Formel (2), (3) oder (4) einer der Formeln (2a), (3a) oder (4a) entspricht, 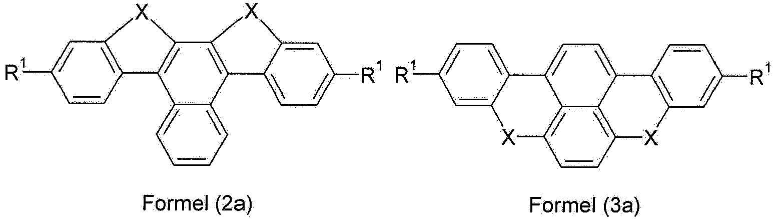 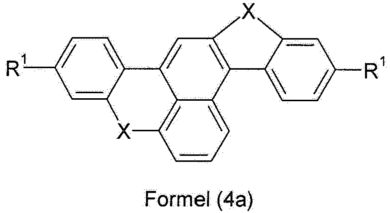 wobei die Symbole X und R¹ dieselbe Bedeutung haben, wie unter Anspruch 1 beschrieben, und die Naphthalingruppe durch einen oder mehrere Reste R¹ substituiert sein kann.

3. Organische Elektrolumineszenzvorrichtung gemäß Anspruch 1 oder 2, **dadurch gekennzeichnet, dass** das Symbol R¹ gleich oder verschieden bei jedem Auftreten für H, F, C(=O)Ar, P(=O)(Ar)₂, -CR²=CR²Ar, eine geradkettige Alkylgruppe mit 1 bis 5 C-Atomen oder eine verzweigte Alkylgruppe mit 3 bis 5 C-Atomen, wobei eine oder mehrere nicht benachbarte CH₂-Gruppen durch -R²C=CR²-, -C≡C- oder -O- ersetzt sein können und wobei ein oder mehrere H-Atome durch F ersetzt sein können, oder eine Arylgruppe mit 6 bis 16 C-Atomen oder eine Heteroarylgruppe mit 2 bis 16 C-Atomen oder eine Spirobifluorengruppe, die jeweils mit einem oder mehreren Resten R² substituiert sein kann, oder eine Kombination aus zwei oder drei dieser Systeme steht, oder dass das Symbol R¹ gleich oder verschieden bei jedem Auftreten für eine Gruppe der Formel (5) oder (6) steht, 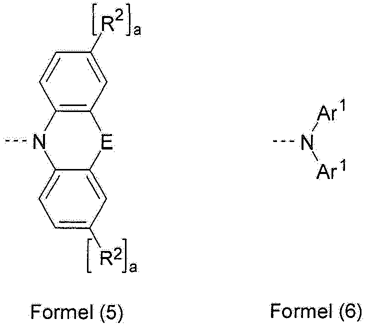 wobei R² die oben aufgeführte Bedeutung hat und weiterhin gilt:
E steht für eine Einfachbindung, O, S, N(R²) oder C(R²)₂;
Ar¹ ist gleich oder verschieden bei jedem Auftreten eine Aryl- oder Heteroarylgruppe mit 5 bis 20 aromatischen Ringatomen oder eine Triarylamingruppe mit 15 bis 30 aromatischen Ringatomen, welche jeweils mit einem oder mehreren Resten R² substituiert sein kann, bevorzugt eine Aryl- oder Heteroarylgruppe mit 6 bis 14 aromatischen Ringatomen oder eine Triarylamingruppe mit 18 bis 22 aromatischen Ringatomen, welche jeweils mit einem oder mehreren Resten R² substituiert sein kann;
a ist bei jedem Auftreten gleich oder verschieden 0 oder 1.

4. Organische Elektrolumineszenzvorrichtung gemäß mindestens einem der vorhergehenden Ansprüche, **dadurch gekennzeichnet, dass** die Verbindung der Formel (2a) entspricht 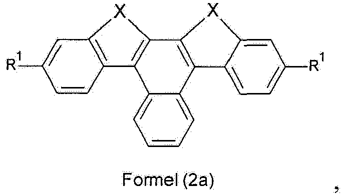 wobei die Naphthalingruppe durch einen oder mehrere Reste R¹ substituiert sein kann.

5. Organische Elektrolumineszenzvorrichtung gemäß mindestens einem der vorhergehenden Ansprüche, **dadurch gekennzeichnet, dass** das Symbol R¹ gleich oder verschieden bei jedem Auftreten für H, F, C(=O)Ar, P(=O)(Ar)₂, -CR²=CR²Ar, eine geradkettige Alkylgruppe mit 1 bis 5 C-Atomen oder eine verzweigte Alkylgruppe mit 3 bis 5 C-Atomen, wobei eine oder mehrere nicht benachbarte CH₂-Gruppen durch -R²C=CR²-, -C≡C- oder -O- ersetzt sein können und wobei ein oder mehrere H-Atome durch F ersetzt sein können, oder eine Arylgruppe mit 6 bis 16 C-Atomen oder eine Heteroarylgruppe mit 2 bis 16 C-Atomen oder eine Spirobifluorengruppe, die jeweils mit einem oder mehreren Resten R² substituiert sein kann, steht.

6. Organische Elektrolumineszenzvorrichtung gemäß mindestens einem der vorhergehenden Ansprüche, **dadurch gekennzeichnet, dass** die mindestens eine Verbindung gemäß Formel (2), (3) oder (4) in einer emittierenden Schicht als Mischung mit mindestens einem phosphoreszierenden Dotanden eingesetzt wird.

## Claims

1. Organic electroluminescent device comprising anode, cathode and at least one emitting layer, **characterised in that** at least one compound of the formula (2), (3) or (4) is employed in an emitting layer as a mixture with at least one fluorescent or phosphorescent dopant, 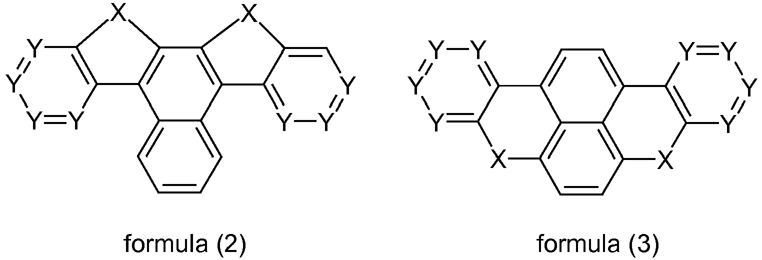 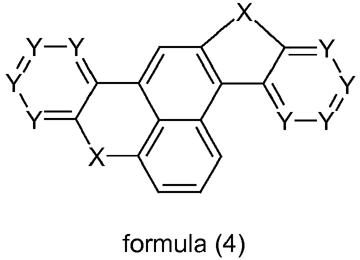 where the following applies to the symbols and indices used:
Y is selected on each occurrence, identically or differently, from CH and CR¹, where at most two groups R¹ may be present per aromatic six-membered ring;
R¹ is on each occurrence, identically or differently, H, F, Cl, Br, I, N(Ar)₂, C(=O)Ar, P(=O)(Ar)₂, S(=O)Ar, S(=O)₂Ar, CR²=CR²Ar, CN, NO₂, Si(R²)₃, B(OR²)₂, a straight-chain alkyl, alkoxy or thioalkoxy group having 1 to 40 C atoms or a branched or cyclic alkyl, alkoxy or thioalkoxy group having 3 to 40 C atoms, which may in each case be substituted by one or more radicals R², where one or more radicals R² may be substituted, where one or more non-adjacent CH₂ groups may be replaced by -R²C=CR²-, -C≡C-, Si(R²)₂, Ge(R²)₂, Sn(R²)₂, C=O, C=S, C=Se, C=NR², P(=O)(R²), SO, SO₂, -NR²-, -O-, -S- or -CONR²- and where one or more H atoms may be replaced by F, Cl, Br, I, CN or NO₂, or an aromatic or heteroaromatic ring system having 5 to 40 aromatic ring atoms, which may in each case be substituted by one or more radicals R², or an aryloxy or heteroaryloxy group having 5 to 40 aromatic ring atoms, which may be substituted by one or more radicals R², or a combination of these systems;
Ar is on each occurrence an aromatic or heteroaromatic ring system having 5-30 aromatic ring atoms, which may be substituted by one or more non-aromatic radicals R¹; two radicals Ar on the same nitrogen atom may also be linked to one another by a single bond or a bridge X;
R² is on each occurrence, identically or differently, H or an aliphatic, aromatic or heteroaromatic hydrocarbon radical having 1 to 20 C atoms; two or more adjacent substituents R² may also form a mono- or polycyclic aliphatic or aromatic ring system with one another;
X is on each occurrence, identically or differently, a divalent bridge selected from C(R¹)₂, C=O, C=NR¹, O, S, S=O, SO₂, N(R¹), P(R¹), P(=O)R¹, C(R₁)₂-C(R¹)₂, C(R¹)₂-C(R¹)₂-C(R¹)₂, C(R¹)₂-O and C(R¹)₂-O-C(R¹)₂;
where the naphthalene group may be substituted by one or more radicals R¹,
**characterised in that** at least one substituent R¹ that contains at least one aryl or heteroaryl group is present.

2. Organic electroluminescent device according to Claim 1, **characterised in that** the compound of the formula (2), (3) or (4) conforms to one of the formulae (2a), (3a) or (4a), 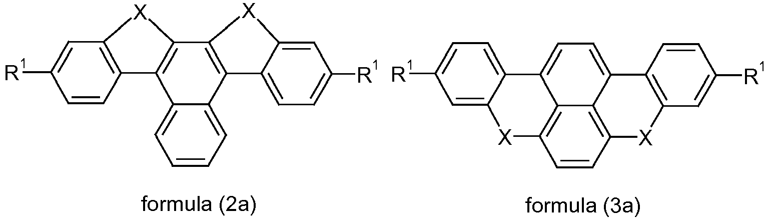 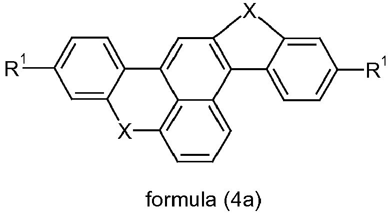 where the symbols X and R¹ have the same meaning as described under Claim 1, and the naphthalene group may be substituted by one or more radicals R¹.

3. Organic electroluminescent device according to Claim 1 or 2, **characterised in that** the symbol R¹ stands, identically or differently on each occurrence, for H, F, C(=O)Ar, P(=O)(Ar)₂, -CR²=CR²Ar, a straight-chain alkyl group having 1 to 5 C atoms or a branched alkyl group having 3 to 5 C atoms, where one or more non-adjacent CH₂ groups may be replaced by -R²C=CR²-, -C≡C- or -O- and where one or more H atoms may be replaced by F, or an aryl group having 6 to 16 C atoms or a heteroaryl group having 2 to 16 C atoms or a spirobifluorene group, which may in each case be substituted by one or more radicals R², or a combination of two or three of these systems, or **in that** the symbol R¹ stands, identically or differently on each occurrence, for a group of the formula (5) or (6), 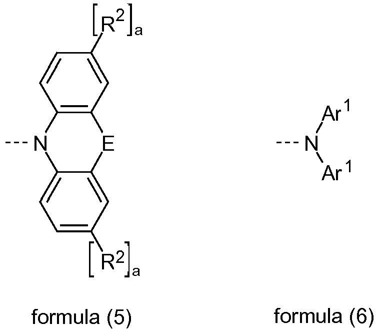 where R² has the meaning indicated above and furthermore:
E stands for a single bond, O, S, N(R²) or C(R²)₂;
Ar¹ is, identically or differently on each occurrence, an aryl or heteroaryl group having 5 to 20 aromatic ring atoms or a tri-arylamine group having 15 to 30 aromatic ring atoms, which may in each case be substituted by one or more radicals R², preferably an aryl or heteroaryl group having 6 to 14 aromatic ring atoms or a triarylamine group having 18 to 22 aromatic ring atoms, which may in each case be substituted by one or more radicals R²;
a is on each occurrence, identically or differently, 0 or 1.

4. Organic electroluminescent device according to at least one of the preceding claims, **characterised in that** the compound conforms to the formula (2a) 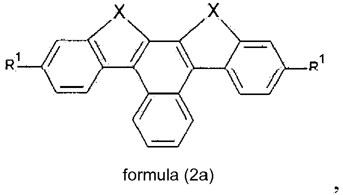 where the naphthalene group may be substituted by one or more radicals R¹.

5. Organic electroluminescent device according to at least one of the preceding claims, **characterised in that** the symbol R¹ stands, identically or differently on each occurrence, for H, F, C(=O)Ar, P(=O)(Ar)₂, -CR²=CR²Ar, a straight-chain alkyl group having 1 to 5 C atoms or a branched alkyl group having 3 to 5 C atoms, where one or more non-adjacent CH₂ groups may be replaced by -R²C=CR²-, -C≡C- or -O- and where one or more H atoms may be replaced by F, or an aryl group having 6 to 16 C atoms or a heteroaryl group having 2 to 16 C atoms or a spirobifluorene group, which may in each case be substituted by one or more radicals R².

6. Organic electroluminescent device according to at least one of the preceding claims, **characterised in that** the at least one compound of the formula (2), (3) or (4) is employed in an emitting layer as a mixture with at least one phosphorescent dopant.

## Revendications

1. Dispositif électroluminescent organique comprenant une anode, une cathode et au moins une couche d'émission, **caractérisé en ce qu'**au moins un composé de la formule (2), (3) ou (4) est utilisé dans une couche d'émission en tant que mélange avec au moins un dopant fluorescent ou phosphorescent, 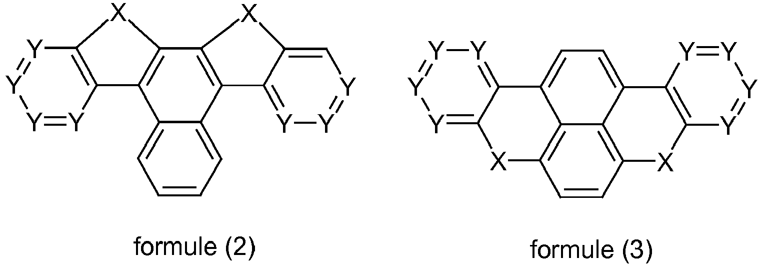 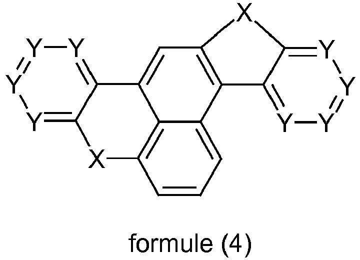 dans lesquelles ce qui suit s'applique aux symboles et aux indices qui sont utilisés :
Y est sélectionné pour chaque occurrence, de manière identique ou différente, parmi CH et CR¹, où au plus deux groupes R¹ peuvent être présents par cycle aromatique à six éléments ;
R¹ est pour chaque occurrence, de manière identique ou différente, H, F, Cl, Br, I, N(Ar)₂, C(=O)Ar, P(=O)(Ar)₂, S(=O)Ar, S(=O)₂Ar, CR²=CR²Ar, CN, NO₂, Si(R²)₃, B(OR²)₂, un groupe alkyle, alcoxy ou thioalcoxy en chaîne droite qui comporte de 1 à 40 atome(s) de C ou un groupe alkyle, alcoxy ou thio-alcoxy ramifié ou cyclique qui comporte de 3 à 40 atomes de C, lequel peut dans chaque cas être substitué par un radical ou par plusieurs radicaux R², où un radical ou plusieurs radicaux R² peut/peuvent être substitué(s), où un ou plusieurs groupe(s) CH₂ non adjacents peut/peuvent être remplacé(s) par-R²C=CR²-, -C≡C-, Si(R²)₂, Ge(R²)₂, Sn(R²)₂, C=O, C=S, C=Se, C=NR², P(=O)(R²), SO, SO₂, -NR²-, -O-, -S- ou -CONR²- et où un ou plusieurs atome(s) de H peut/peuvent être remplacé(s) par F, Cl, Br, I, CN ou NO₂, ou un système de cycle aromatique ou hétéroaromatique qui comporte de 5 à 40 atomes de cycle aromatique, lequel peut dans chaque cas être substitué par un radical ou par plusieurs radicaux R², ou un groupe aryloxy ou hétéroaryloxy qui comporte de 5 à 40 atomes de cycle aromatique, lequel peut être substitué par un radical ou par plusieurs radicaux R², ou une combinaison de ces systèmes ;
Ar est pour chaque occurrence un système de cycle aromatique ou hétéroaromatique qui comporte de 5 à 30 atomes de cycle aromatique, lequel peut être substitué par un radical ou par plusieurs radicaux R¹ non aromatique(s) ; deux radicaux Ar sur le même atome d'azote peuvent également être liés l'un à l'autre par une liaison simple ou par un pont X ;
R² est pour chaque occurrence, de manière identique ou différente, H ou un radical hydrocarbone aliphatique, aromatique ou hétéroaromatique qui comporte de 1 à 20 atome(s) de C ; deux substituants R² adjacents ou plus peuvent également former un système de cycle aliphatique ou aromatique mono- ou polycyclique l'un avec l'autre ou les uns avec les autres ; et
X est pour chaque occurrence, de manière identique ou différente, un pont divalent qui est sélectionné parmi C(R¹)₂, C=O, C=NR¹, O, S, S=O, SO₂, N(R¹), P(R¹), P(=O)R¹, C(R¹)₂-C(R¹)₂, C(R¹)₂-C(R¹)₂-C(R¹)₂, C(R¹)₂-O et C(R¹)₂-O-C(R¹)₂ ;
où le groupe naphtalène peut être substitué par un radical ou par plusieurs radicaux R¹ ;
**caractérisé en ce qu'**au moins un substituant R¹ qui contient au moins un groupe aryle ou hétéroaryle est présent.

2. Dispositif électroluminescent organique selon la revendication 1, **caractérisé en ce que** le composé de la formule (2), (3) ou (4) est conforme à l'une des formules (2a), (3a) ou (4a), 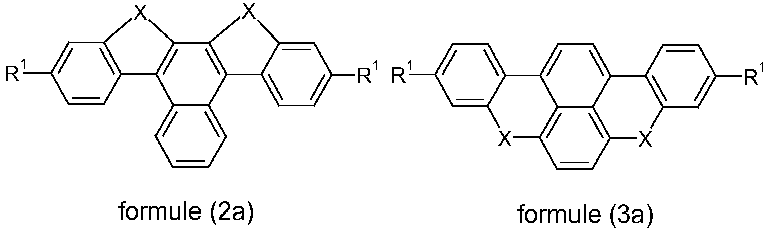 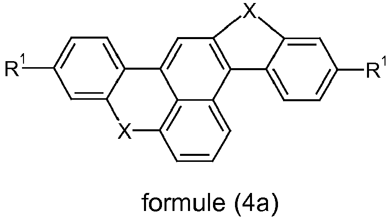 dans lesquelles les symboles X et R¹ présentent la même signification que celle qui a été décrite selon la revendication 1, et le groupe naphtalène peut être substitué par un radical ou par plusieurs radicaux R¹.

3. Dispositif électroluminescent organique selon la revendication 1 ou 2, **caractérisé en ce que** le symbole R¹ représente, de manière identique ou différente pour chaque occurrence, H, F, C(=O)Ar, P(=O)(Ar)₂, -CR²=CR²Ar, un groupe alkyle en chaîne droite qui comporte de 1 à 5 atome(s) de C ou un groupe alkyle ramifié qui comporte de 3 à 5 atomes de C, où un ou plusieurs groupe(s) CH₂ non adjacents peut/peuvent être remplacé(s) par -R²C=CR²-, -C≡C- ou -O- et où un ou plusieurs atome(s) de H peut/peuvent être remplacé(s) par F, ou un groupe aryle qui comporte de 6 à 16 atomes de C ou un groupe hétéroaryle qui comporte de 2 à 16 atomes de C ou un groupe spirobifluorène, lequel peut dans chaque cas être substitué par un radical ou par plusieurs radicaux R², ou une combinaison de deux ou trois de ces systèmes, ou **en ce que** le symbole R¹ représente, de manière identique ou différente pour chaque occurrence, un groupe de la formule (5) ou (6), 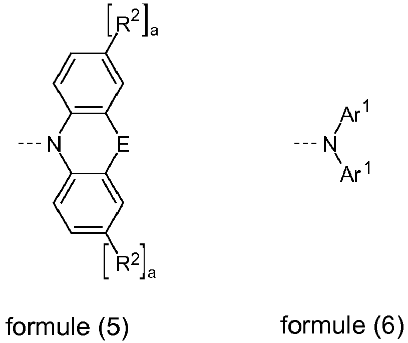 dans lesquelles R² présente la signification qui a été indiquée ci-avant et en outre :
E représente une liaison simple, O, S, N(R²) ou C(R²)₂ ;
Ar¹ est, de manière identique ou différente pour chaque occurrence, un groupe aryle ou hétéroaryle qui comporte de 5 à 20 atomes de cycle aromatique ou un groupe triarylamine qui comporte de 15 à 30 atomes de cycle aromatique, lequel peut dans chaque cas être substitué par un radical ou par plusieurs radicaux R², de préférence un groupe aryle ou hétéroaryle qui comporte de 6 à 14 atomes de cycle aromatique ou un groupe triarylamine qui comporte de 18 à 22 atomes de cycle aromatique, lequel peut dans chaque cas être substitué par un radical ou par plusieurs radicaux R² ; et
a est pour chaque occurrence, de manière identique ou différente, 0 ou 1.

4. Dispositif électroluminescent organique selon au moins l'une des revendications qui précèdent, **caractérisé en ce que** le composé est conforme à la formule (2a) 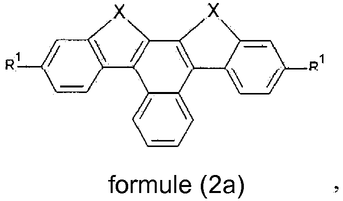 dans laquelle le groupe naphtalène peut être substitué par un radical ou par plusieurs radicaux R¹.

5. Dispositif électroluminescent organique selon au moins l'une des revendications qui précèdent, **caractérisé en ce que** le symbole R¹ représente, de manière identique ou différente pour chaque occurrence, H, F, C(=O)Ar, P(=O)(Ar)₂, -CR²=CR²Ar, un groupe alkyle en chaîne droite qui comporte de 1 à 5 atome(s) de C ou un groupe alkyle ramifié qui comporte de 3 à 5 atomes de C, où un ou plusieurs groupe(s) CH₂ non adjacents peut/peuvent être remplacé(s) par -R²C=CR²-, -C≡C- ou -O- et où un ou plusieurs atome(s) de H peut/ peuvent être remplacé(s) par F, ou un groupe aryle qui comporte de 6 à 16 atomes de C ou un groupe hétéroaryle qui comporte de 2 à 16 atomes de C ou un groupe spirobifluorène, lequel peut dans chaque cas être substitué par un radical ou par plusieurs radicaux R².

6. Dispositif électroluminescent organique selon au moins l'une des revendications qui précèdent, **caractérisé en ce que** l'au moins un composé de la formule (2), (3) ou (4) est utilisé dans une couche d'émission en tant que mélange avec au moins un dopant phosphorescent.
